# EUROPÄISCHE PATENTANMELDUNG

(11) **EP 2 992 851 A1**
(43) Veröffentlichungstag der Anmeldung: **09.03.2016**
(21) Anmeldenummer: 15183986.7
(22) Anmeldetag: 06.09.2015
(51) Int. Cl.: A61B 50/30

(54) **VERPACKUNGSSYSTEM FÜR ASEPTISCH ZU PRÄSENTIERENDE MEDIZINISCHE INSTRUMENTE SOWIE VERFAHREN UNTER VERWENDUNG DES VERPACKUNGSSYSTEMS**

(30) Priorität: 08.09.2014 DE 102014112911
(71) Anmelder: Bröker, Natalija, 41066 Mönchengladbach (DE)
(72) Erfinder: Bröker, Natalija, 41066 Mönchengladbach (DE)
(74) Vertreter: Bonsmann, Joachim Bernhard

(57) **Zusammenfassung**

Die Erfindung betrifft ein Verpackungssystem für ggf. in einem oder mehreren Siebkörben aufbewahrte, aseptisch zu präsentierende medizinische Instrumente, mit wenigstens einem vliesartigen Flächengebilde (10), in welches die medizinischen Instrumente bzw. der diese aufbewahrende wenigstens eine Siebkorb einschlagbar sind, und einem aus einem Bodenteil (12, 12') und einem zu öffnenden Deckelteil (14, 14') bestehenden Kartonelement oder kartonförmigen Element, das zur geschlossenen Aufbewahrung der in das wenigstens eine vliesartiges Flächengebilde (10) eingeschlagenen Instrumente ausgebildet ist, wobei das Verpackungssystem insgesamt sterilisierbar ausgebildet ist. Ferner wird ein Verfahren unter Verwendung dieses Verpackungssystems beschrieben.

## Beschreibung

Die vorliegende Erfindung betrifft ein Verpackungssystem für aseptisch zu präsentierende medizinische Instrumente sowie ein Verfahren zum Sterilitätserhalt während Lagerung und Transport sowie zur aseptischen Präsentation medizinischer Instrumente mittels eines derartigen Verpackungssystems.

Medizinische, bestimmungsgemäß steril zur Anwendung kommende Instrumente (beispielsweise chirurgische Instrumente) müssen, sofern es sich nicht um Einwegartikel handelt, bekanntermaßen vor Verwendung am Patienten umfassend desinfiziert und sterilisiert werden. Dies geschieht meistens in einer speziellen Sterilisationsabteilung.

Im Anschluss der Sterilisation muss gewährleistet werden, dass die Sterilität bei Lagerung und beim Transport zum Verwendungsort aufrechterhalten bleibt. Dies geschieht im Stand der Technik durch verschiedene sog. Verpackungssysteme, die in der Regel ein sog. Sterilbarrieresystem zur Aufrechterhaltung der Keimfreiheit und eine umgebende Schutzverpackung zur Abschirmung gegenüber mechanischen Einflüssen aufweisen.

Bei Verwendung der Instrumente, z.B. im Rahmen einer Operation, werden die nicht sterilen Komponenten des Verpackungssystems von einer entsprechend qualifizierten, nicht steril gewaschenen Person (sog. "Springer") entfernt und eine steril gewaschene Person (die sog. instrumentierende Person) entnimmt die sterilen Instrumente. Dieser Vorgang wird auch als "aseptische Präsentation" bezeichnet.

Im Stand der Technik sind verschiedene Verpackungssysteme bekannt.

Viele aufzubewahrende Instrumente werden unmittelbar in metallischen Siebkörben (auch als Siebschale bezeichnet) aufbewahrt und dort nach Anwendungsgebieten geordnet. Diese gefüllten Siebschalen bilden sog. Instrumentensets. Da die meisten Instrumente ebenfalls aus Metall hergestellt sind, beträgt das Gewicht derartiger gefüllter Siebkörbe häufig mehr als 10 kg, auch wenn normgemäß maximal 10 kg vorgesehen sind.

Ferner kann nicht ganz ausgeschlossen werden, dass Instrumente aus den Siebkörben hervorstehen bzw. z.B. Kanülen durch die Siebkorböffnungen hindurchtreten.

Andere Instrumente werden in Klarsichtbeuteln einzeln verpackt oder ohne weitere Umverpackung in den weiter unten diskutierten Verpackungssystemen aufbewahrt (z.B. Kochsalz-/Waschschüsseln).

Ein häufig in Krankenhäusern anzutreffendes Verpackungssystem sind sog. Container. Dabei handelt es sich um verschließbare und geschlossene Aluminiumbehälter zur Aufnahme der Siebkörbe oder anders verpackter Instrumente mit einem speziellen Filter- und Ventilsystem (in der Regel unter Verwendung von Einwegfiltern) zur Kommunikation mit der Umgebung. Durch dieses Filter- und Ventilsystem soll gewährleistet werden, dass während des Sterilisationsvorganges die Luft aus dem Behälter abgepumpt werden kann (Vakuum) und Sattdampf eintreten kann, der Behälter aber andererseits unter normalen Umgebungsbedingungen (d.h. Lagerung und Transport) keimdicht verschlossen ist.

Die entsprechenden Behälter sind zwar recht praktisch und bieten den Vorteil eines Mehrwegproduktes. Andererseits sind die Anschaffungskosten recht hoch und die Filter- und Ventilsysteme bedürfen einer regelmäßigen Wartung, die einen zusätzlichen Kostenfaktur darstellt und erfahrungsgemäß häufig sogar vernachlässigt wird. Dementsprechend gibt es durchaus Container, die sich seit vielen Jahren ohne Wartung im Einsatz befinden und bei denen nicht sicher ist, ob die Keimdichtheit unter Umgebungsbedingungen bzw. die Dampf- oder Vakuumdurchlässigkeit während der Sterilisation noch wirklich einwandfrei gewährleistet ist, zumal man dies auch nicht ohne aufwändige Prüfstände feststellen kann. Ohne zusätzliche Innenverpackung (siehe unten) unterliegt die aseptische Präsentation der Instrumente bei diesen Verpackungen somit gewissen Kontaminationsrisiken.

Ein weiterer Nachteil des Containersystems besteht darin, dass eine wirklich zuverlässige Kontrolle der Unversehrtheit eines sterilisierten Containers, d.h., ob dieser schon einmal geöffnet war und daher unsteril geworden ist, letztlich kaum möglich ist. Es existieren zwar spezielle Versiegelungs- und Verplombungssysteme, deren Nachweissicherheit in der Praxis jedoch unbefriedigend ist.

Darüber hinaus müssen die Container nach jedem Gebrauch in einer speziellen Maschine gereinigt und desinfiziert werden. Daraus entstehen nicht unerhebliche Kosten für deren Anschaffung, Betriebsmittel und Personal.

Ein bei sachgemäßer Handhabung relativ sicheres Verpackungssystem basiert auf dem Einschlagen der Siebkörbe bzw. der Instrumente in vliesartige Flächengebilde, die eine sehr gute Dampfdurchlässigkeit für den Sterilisiervorgang aufweisen und bei sachgerechter Handhabung, sofern keine Beschädigungen (Perforationen) durch die Instrumente auftreten, die Sterilität dauerhaft sicherstellen können. Derartige Beschädigungen, die grundsätzlich gelegentlich unvermeidlich sind, können durch visuelle Inspektion der Vliese zuverlässig erkannt werden. Bei den Vliesen handelt es sich um Einwegartikel.

Je nach Vorgehensweise unterscheidet man bei den vliesbasierten Verpackungssystemen zwischen Weichverpackungen mit zwei getrennten oder miteinander verbundenen Bögen, in die die Siebkörbe bzw. nicht in einem Siebkorb aufbewahrte Instrumente in einem einzigen Verpackungsschritt eingeschlagen werden. Alternativ ist auch die sequentielle Verpackung mit zwei separaten Bögen bekannt, die zwar arbeitsaufwendiger ist, jedoch u.a. eine zuverlässigere aseptische Präsentation ermöglicht.

Wenn Vliesverpackungen als Sterilbarriere und Transportverpackung gleichzeitig genutzt werden, sind Perforationen der Vliese durch die schweren Siebkörbe und die wie erwähnt aus diesen teilweise herausragenden Instrumente oder beim Transport relativ häufig. Auch ist die Stapelbarkeit suboptimal. Um einen sicheren Transport und Lagerung zu gewährleisten, müssen spezielle Drahtkörbe zum Einsatz kommen, deren Anschaffung sehr kostenintensiv ist.

Der vorliegenden Erfindung liegt die Aufgabe zugrunde, ein Verpackungssystem der eingangs genannten Art zu schaffen, das hohe Zuverlässigkeit mit einem guten mechanischen Schutz vor Perforation bei grundsätzlicher Stapelbarkeit gewährleistet und ein optimalen Preis-/Leistungsverhältnis bietet.

Die Lösung der vorgenannten Aufgabe erfolgt mittels eines Verpackungssystems mit den Merkmalen des Patentanspruchs 1.

Vorteilhafte Ausgestaltungen sind in den abhängigen Patentansprüchen beschrieben.

Ferner wird zur Lösung der vorgenannten Aufgabe ein Verfahren zum Sterilitätserhalt während Lagerung und Transport sowie zur aseptischen Präsentation medizinischer Instrumente mittels eines entsprechenden Verpackungssystems mit den Verfahrensschritten gemäß Patentanspruch 14 vorgeschlagen.

Erfindungsgemäß weist ein Verpackungssystem für ggf. in einem oder mehreren Siebkörben aufbewahrte, aseptisch zu präsentierende medizinische Instrumente wenigstens ein vliesartiges Flächengebilde, in welches die medizinischen Instrumente bzw. der diese aufbewahrende wenigstens eine Siebkorb einschlagbar sind, und ein aus einem Bodenteil und einem zu öffnenden Deckelteil bestehendes Kartonelement oder kartonförmiges Element auf, das zur geschlossenen Aufbewahrung der in das wenigstens eine vliesartiges Flächengebilde eingeschlagenen Instrumente bzw. des wenigstens einen Siebkorbs ausgebildet ist, wobei das Verpackungssystem insgesamt sterilisierbar ausgebildet ist.

Durch die Kombination wenigstens eines vliesartigen Flächengebildes, das im Rahmen der Erfindung das Sterilbarrieresystem bildet, und einer kartonartigen Umverpackung, die als Transportverpackung fungiert, wird ein im Handling erheblich robusteres System als bei den reinen vliesbasierten Weichverpackungssystemen realisiert.

Gleichzeitig werden die wesentlichen Nachteile der Containersysteme vermieden, denn die Kartonverpackungen sind preiswert und als Einwegverpackungen realisierbar, so dass sich keine Fragen hinsichtlich Langzeitstabilität, Wartung und Aufbereitung stellen. Da Papier ein sehr gut recyclebarer Rohstoff darstellt, der in Krankenhäusern sowieso anderweitig separat gesammelt wird, ist auch die Umweltbilanz dieser Lösung positiv, zumal die benutzten Kartonelemente nicht als medizinischer Sondermüll entsorgt werden müssen.

Bei dem erfindungsgemäßen Verpackungssystem werden Siebkörbe mit den medizinischen Instrumenten (ggf. auch mehrere Siebkörbe nebeneinander oder übereinander gestapelt) oder die siebkorblos oder anderweitig verpackten Instrumente in ein oder mehrere vliesartige Flächengebilde so eingeschlagen, dass hierdurch bereits eine Sterilbarriere gegeben ist. Die entsprechende Vorgehensweise ist bekannt und in Normen und Richtlinien zum Umgang mit Weichverpackungen beschrieben.

Anschließend wird die Einheit in das Kartonelement gestellt (sofern das Einschlagen nicht bereits auf dem Bodenteil des Kartonelements erfolgte) und mit dem Deckel verschlossen und diese Einheit dann sterilisiert.

Um dies zu gewährleisten, muss das verwendete Kartonelement sterilisierbar sein, d.h., es muss vom Kartonmaterial her den Sterilitätsbedingungen standhalten und auch so wenig Wasser aufnehmen, dass dieses während des Sterilisierungsvorganges wieder verdunsten kann. Gleichzeitig muss die Dampfdurchlässigkeit so gut sein, dass auch das innenliegende Sterilisiergut optimal erreicht wird. Sollte die Dampfdurchlässigkeit nicht ausreichen, so können in dem Kartonelement Öffnungen - ggf. mit einem Filtermaterial verschlossen - vorgesehen sein, die als zusätzlicher Dampfeinlass wirken. Damit durch diese Öffnungen unter Lager- und Transportbedingungen kein Staub od. dgl. eindringt, können diese Öffnungen labyrinthisch abgeschirmt bzw. verdeckt ausgebildet sein. Beispielsweise könnten entsprechende Öffnungen im oberen Bereich der Seitenwände des Bodenteils vorgesehen sein, die dann von dem Kragen des Deckelteils abgedeckt werden.

Neben den "klassischen" Kartonelementen, die primär aus einem papierartigen Material bestehen, das je nach gewünschten Oberflächen- und Wasseraufnahmeeigenschaften ggf. noch mit einer Folie und/oder einer Beschichtung versehen sein kann, können im Rahmen der Erfindung auch als "kartonförmig" bezeichnete Elemente eingesetzt werden, die eine typische Karton- oder Schachtelform aufweisen, jedoch primär nicht aus einem Papiermaterial bestehen, sondern aus einem anderen flexiblen, kartonartigen Material, beispielsweise einem Kunststoffmaterial (nicht jedoch aus einem starren Material, also nicht aus einem Metallblech wie bekannte Container). Hierfür kämen beispielsweise Polymerfolien oder dünne geschäumte Materialien infrage, z.B. Polystyrol in geschäumten oder ungeschäumten Zustand.

In einer bevorzugten Ausführungsform kann das Kartonelement als zweiteilige Faltschachtel mit ineinander stülpbarem Bodenteil und Deckelteil jeweils mit umlaufenden Seitenkrägen ausgebildet sein.

Alternativ kann das Kartonelement als einteiliger Faltkarton mit Boden-, Deckel- und Seitenwandteilen ausgebildet ist, wobei der Deckelteil scharnierartig aufklappbar mit einem Seitenwandteil verbunden ist.

Um eine flexible Anpassbarkeit an die Dimension der aufzubewahrenden Instrumente bzw. Siebkörbe mit Instrumenten zu erzielen, kann in einer Ausführungsform das das Kartonelement oder kartonförmige Element aus zwei ineinander gesteckten, an einer Seite offenen Teilen bestehen, so dass das Element in seiner Längs- oder Breitenerstreckung teleskopierbar ist. Damit kann insbesondere für sog. "Leihinstrumente", die manchmal ungewöhnlich große Erstreckungen in einer Dimension aufweisen, ein flexibles Verpackungssystem geschaffen werden, ohne dass eine Vielzahl verschiedener Kartongrößen vorgehalten werden müsste. Alternativ kann man natürlich derartige Instrumente in einem wesentlich größeren Karton sterilisieren. Dies ist aber sowohl für den Sterilisiervorgang, den Platzbedarf im Sterilisator und den Platzbedarf für die Aufbewahrung suboptimal,

Falls doch ein für den Inhalt etwas zu großes Karton- oder kartonförmiges Element verwendet wird, können in diesem selektiv ausklappbare bzw. einstellbare Begrenzungs- oder Fixierungselemente vorgesehen sein (z.B. hochklappbare vorgestanzte Laschen in einer inneren Kartonlage in einem Boden mit zwei Kartonlage, verstellbare Kartonbänder etc.), mittels derer ein Umherrutschen der Instrumente bzw. Siebkörbe innerhalb des Karton- oder kartonförmigen Elements beim Transport vermieden oder vermindert werden kann, wodurch den Sterilzustand gefährdende Beschädigungen des Karton- oder kartonförmigen Elements und/oder des vliesartigen Flächengebildes bei einem Umherrutschen des Inhalts vermieden werden können.

In einer bevorzugten Ausgestaltung kann das wenigstens eine vliesartige Flächengebilde an dem Bodenteil wenigstens in einem Teilbereich innenseitig fixiert sein. Hierdurch wird das korrekte Einschlagen der Siebkörbe bzw. der Instrumente vereinfacht, da das Vlies unmittelbar korrekt zentriert an dem Bodenteil angebracht ist.

In einer bevorzugten Ausführungsform ist ein einziges vliesartiges Flächengebilde vorgesehen, das rechteckförmig ausgebildet ist, und vorzugsweise in einem Winkel von etwa 45° zur Ausrichtung des Bodens an diesem im Mittenbereich der innenseitigen Bodenfläche fixiert ist. Alternativ zu der erwähnten 45°-Fixierung kann das vliesartige Flächengebilde aber auch parallel zu den Kartonkanten angeordnet sein.

Weiterhin kann das vliesartige rechteckförmige Flächengebilde alternativ auch ohne Verbindung mit dem Bodenteil dem Kartonelement in Form eines Verpackungssets lose beigefügt sein.

Ferner können Versiegelungsmittel, insbesondere Klebestreifen, vorgesehen sein, mittels derer ein einmaliges Öffnen des Kartonelements nach Anbringen der Versiegelungsmittel sicher dauerhaft erkannt werden kann. Da bei ausreichend klebestarken Klebebändern der Karton bei Abziehen derselben beschädigt wird und dieser nicht wiederverwendet wird, ist eine zuverlässige Versiegelung mit einfachen Mitteln möglich.

Ferner können an dem Kartonelement und/oder dem wenigstens einen vliesartigen Flächengebilde Indikatormittel zur dauerhaften Anzeige eines einmal vorgenommenen Sterilisationsvorganges vorgesehen sein. Derartige Indikatormittel, die sich durch den Sterilisationsvorgang verfärben bzw. entfärben, sind im Stand der Technik bereits bekannt. In Verbindung mit dem Versiegelungsmittel wird hierdurch ein hoher Grad an Verwendungssicherheit gewährleistet.

Weiterhin wird im Rahmen der Erfindung ein Kartonelement beansprucht, das zur Verwendung bei einem Verpackungssystem nach einem der vorhergehenden Ansprüche ausgebildet ist. Hierzu ist das Kartonelement bevorzugt auf die Größe der aufzunehmenden Siebkörbe dimensionsmäßig angepasst; vorzugsweise so, dass der Kartondeckel auf der Oberkante des Siebkorbs oder der übereinander gestapelten Siebkörbe aufliegt und so eine gute Stapelbarkeit der Kartons ermöglicht.

Bei einem erfindungsgemäßen Verfahren zum Sterilitätserhalt während Lagerung und Transport sowie aseptischer Präsentation medizinischer Instrumente mittels eines Verpackungssystems sind folgende Schritte vorgesehen:
a) Bereitstellen der noch unsterilisierten medizinischen Instrumente, ggf. in wenigstens einem Siebkorb;
b) Einschlagen der medizinischen Instrumente bzw. des wenigstens einen Siebkorbs in wenigstens ein vliesartiges Flächengebilde, so dass diese vollständig von dem Flächengebilde umhüllt sind,
c) Einpacken der eingeschlagenen medizinischen Instrumente bzw. des wenigstens einen Siebkorbs in ein geschlossenes Kartonelement mit einem Bodenteil und einem Deckelteil;
d) Sterilisieren des geschlossenen Kartonelements mit Inhalt;
e) Lagerung des sterilisierten Kartonelements und Transport zum Einsatzort;
f) am Einsatzort Öffnen des Deckelteils und Eröffnung des vliesartiges Flächengebilde sowie Entnahme der medizinischen Instrumente.

Der letztgenannte Schritt (f) stellt die aseptische Präsentation dar, wobei für das Öffnen des (in der Regel nicht mehr sterilen) Deckelteils des Kartonelements der sog. Springer in einem OP-Saal zuständig ist. Da die Kartonelemente nicht luftdicht sind, ist auch die Außenseite des vliesförmigen Flächengebildes nicht mehr notwendigerweise steril, weshalb diese auch von dem Springer eröffnet wird, ohne dass dieser die Innenseite sowie die Instrumente berührt. Dabei wird die Unversehrtheit des Flächengebildes (z.B. auf Perforationen) visuell überprüft. Schließlich erfolgt der Zugriff auf die sterilen Instrumente durch die sog. Instrumentierende (steril gewaschen).

In einer bevorzugten Ausgestaltung kann zwischen Schritt c) und d) eine Versiegelung mit einem Klebebandstreifen erfolgen.

Die Erfindung nachfolgend anhand der Zeichnungen beispielhaft näher erläutert. Es zeigen:
- Figur 1a-c: eine schematische Darstellung einer ersten Ausführungsform der Erfindung unter Verwendung eines Stülpdeckelkartons;
- Figur 2a,b: eine schematische Darstellung einer zweiten Ausführungsform der Erfindung unter Verwendung eines Faltkartons; und
- Figur 3: eine schematische Darstellung eines Ausführungsform der Erfindung mit einem längenverstellbaren Faltkarton.

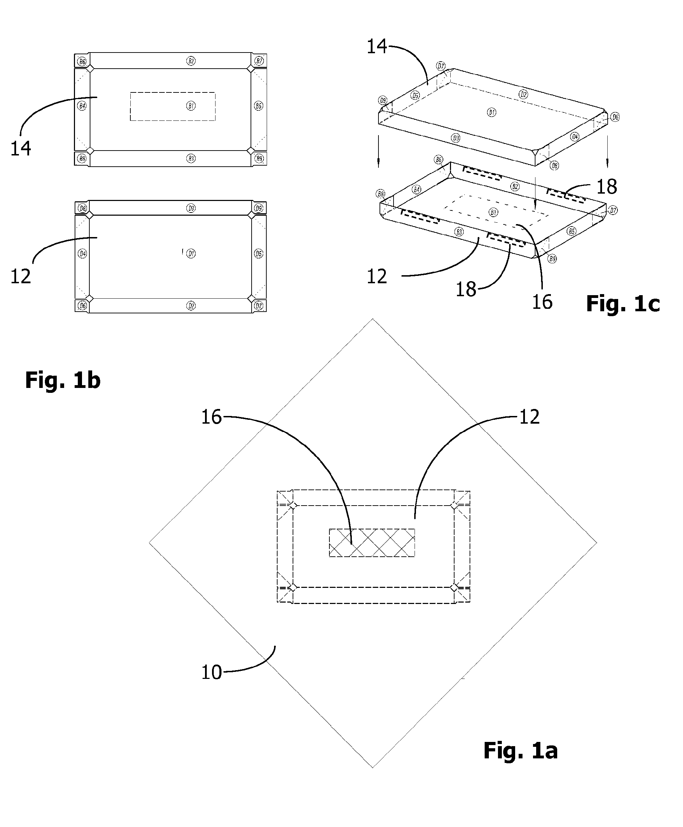

Gemäß Figur 1a ist ein Bodenteil 12 eines Kartonelements zur Verwendung bei der vorliegenden Erfindung in Abwicklung dargestellt, d.h., die Seitenwände befinden sich noch nicht in der für die Verwendung aufgestellten Form. Der aufgestellte Zustand ist aus Figur 1 c ersichtlich.

Typische Abmessungen eines derartigen Kartonelements betragen ca. 31 x 61 x 5,5 cm, was auf gängige Maße der verwendeten Siebkörbe (nicht dargestellt) zuzüglich Raum für das umgebende Vlies abgestimmt ist. Letzteres Vlies 10 ist in der dargestellten Ausführungsform über eine Klebefläche 16 mittig an dem Bodenteil 12 fixiert, und zwar in einem Winkel von 45° zu den Seiten des Kartonelements. Diese Vorfixierung erleichtert ein korrektes Einschlagen des Vlieses um den zu sterilisierenden Siebkorb bzw. um die nebeneinander bzw. - bei entsprechender Kartonhöhe auch übereinander stapelbaren - zu sterilisierenden Siebkörbe. Die Vorfixierung kann leicht lösbar ausgebildet sein oder sich während des Sterilisiervorganges von selbst lösen, um ein leichtes Herausnehmen und eine vereinfachte Materialtrennung beim Recycling zu ermöglichen.

Typische Abmessungen eines derartigen, im Beispiel quadratischen Vlieses 10 betragen - abgestimmt auf die vorgenannte beispielhafte Dimensionierung des Kartonelements - z.B. ca. 120 x 120 cm.

In einer nicht dargestellten Ausführungsform ist das Vlies 10 nicht an dem Bodenteil 12 vorfixiert, sondern wird dem Kartonelement separat beiliegend als Set vertrieben.

Weiterhin ist gemäß den Figuren 1 a-c ein Deckelteil 14 vorgesehen, das in der Art eines Stülpdeckels die Seitenwände des Bodenteils 12 umgibt. Höhenvariationen in den in dem Kartonelement befindlichen Siebkörben (nicht dargestellt) können durch das Deckelteil in bestimmten Grenzen ausgeglichen werden; wodurch, sofern das Deckelteil auf den Siebkörben aufliegt, eine gute Stapelbarkeit gegeben ist.

In den Darstellungen von Figur 1b und 1c sind die einzelnen Teile der Abwicklungen der Kartonelemente mit Nummern in Kreisen gekennzeichnet.

Das verwendete Kartonmaterial ist sterilisierbar ausgebildet. Falls die Dampfdurchlässigkeit nicht ausreicht, um eine vollständige Sterilisierung des Inhalts zu gewährleisten, können zusätzliche Öffnungen 18 vorgesehen sein, die jedoch bevorzugt so verdeckt bzw. labyrinthisch angeracht sind, dass ein Eindringen von Staub etc. weitgehend unterbunden wird, ggf. können auch Filterpapiere od. dgl. eingelassen sein. Im dargestellten Ausführungsbeispiel gemäß Figur 1b werden die Öffnungen 18 im Bodenteil 12 durch das Deckelteil 14 verdeckt. Um dennoch eine ausreichende Dampfpassage zu erreichen, kann das Deckelteil ein leichtes Übermaß aufweisen.

Das Kartonelement kann zusätzlich mit einer Folie kaschiert sein oder als "kartonförmiges" Element überwiegend aus einem nicht-papierartigen Material, wie einem Polymer-Kunststoff, insbesondere Polystyrol (geschäumt oder nicht geschäumt) hergestellt sein.

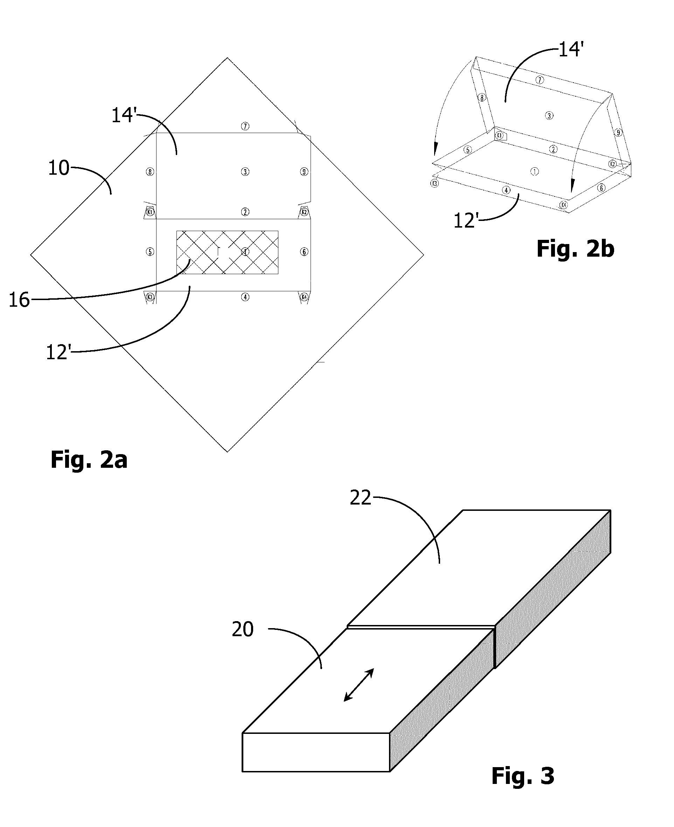

In den Figuren 2a und 2b ist eine alternative zweite Variante eines im Rahmen der Erfindung verwendbaren Kartonelements beispielhaft dargestellt, wobei es sich um einen Faltschachteilkarton mit von Vornherein scharnierartig verbundenem Bodenteil 12' und Deckelteil 14' handelt. Auch hier kann das Vlies 10 entweder über einen Bereich 16 vorfixiert sein oder separat beigelegt werden.

Zur Verwendung werden (nicht dargestellte) Siebkörbe in beiden vorbeschriebenen Ausführungsformen mit zu sterilisierenden Instrumentensets zunächst in das Vlies 10 in der für Weichverpackungen vorgeschriebenen Weise eingeschlagen, ggf. mit dem Bodenteil 12 als Unterlage oder ohne Unterlage. Dann werden hierüber Boden- und Deckelteil geschlossen und die Einheit ggf. mit einem Klebeband oder einem Aufkleber versiegelt. Nach der Behandlung im Sterilisator kann die Einheit gelagert und zur letztlichen Verwendung werden und dort - wie vorstehend bereits beschrieben - aseptisch präsentiert werden.

Figur 3 zeigt schematisch eine Ausführungsform eines Karton- oder kartonförmigen Elements, bei dem dieses aus zwei ineinander gesteckten, teleskopierbaren Teilen 20 und 22 besteht, in der Art eines Stülpdeckelkartons, jedoch dies in der Längs- oder Breitendimension. Hierdurch kann das Kartonelement in einer Dimension (z.B. der Längsdimension) flexibel an den aufzubewahrenden Inhalt angepasst werden, vgl. den Doppelpfeil in Figur 3. Dabei werden die beiden Teile 20, 22 nach dem Einlegen des Inhalts und dem Einstellen der passenden Länge gegeneinander fixiert (z.B. durch ein Klebeband oder eine Lasche). Zur aseptischen Präsentation wird dann der Deckel der beiden Teile 20, 22 mittels entsprechender (nicht dargestellter) Perforationslaschen entfernt, so dass der Zugriff von oben erfolgen kann.

## Patentansprüche

1. Verpackungssystem für ggf. in einem oder mehreren Siebkörben aufbewahrte, aseptisch zu präsentierende medizinische Instrumente,
**gekennzeichnet durch**
wenigstens ein vliesartiges Flächengebilde (10), in welches die medizinischen Instrumente und/oder der diese aufbewahrende wenigstens eine Siebkorb einschlagbar sind, und
ein aus einem Bodenteil (12, 12') und einem zu öffnenden Deckelteil (14, 14') bestehendes Karton- oder kartonförmiges Element, das zur geschlossenen Aufbewahrung der in das wenigstens eine vliesartiges Flächengebilde (10) eingeschlagenen Instrumente und/oder des wenigstens einen Siebkorbs ausgebildet ist, wobei das kartonförmige Element aus einem nicht papierbasierten, gleichwohl kartonartig flexiblen Material besteht,
wobei das Verpackungssystem insgesamt sterilisierbar ausgebildet ist.

2. Verpackungssystem nach Anspruch 1,
**dadurch gekennzeichnet, dass**
das sterilisierbare kartonförmige Element im Wesentlichen aus einem Kunststoff, insbesondere aus einem Polymer, bevorzugt einem Polystyrol in geschäumten oder ungeschäumten Zustand, gebildet ist.

3. Verpackungssystem nach Anspruch 1,
**dadurch gekennzeichnet, dass**
das sterilisierbare Kartonelement aus einem Papiermaterial, das zusätzlich innen- und/oder außenseitig mit einer Kunststoff- oder einer Metallfolie kaschiert sein kann, gebildet ist.

4. Verpackungssystem nach einem der Ansprüche 1 bis 3,
**dadurch gekennzeichnet, dass**
das Kartonelement oder kartonförmige Element als zweiteilige Faltschachtel mit ineinander stülpbarem Bodenteil (12) und Deckelteil (14) jeweils mit umlaufenden Seitenkrägen ausgebildet ist.

5. Verpackungssystem nach einem der Ansprüche 1 bis 3,
**dadurch gekennzeichnet, dass**
das Kartonelement oder kartonförmige Element als einteiliger Faltkarton mit Boden- (12'), Deckel- (14') und Seitenwandteilen ausgebildet ist, wobei das Deckelteil (14') scharnierartig aufklappbar mit einem Seitenwandteil verbunden ist.

6. Verpackungssystem nach einem der Ansprüche 1 bis 5,
**dadurch gekennzeichnet, dass**
das Kartonelement oder kartonförmige Element aus zwei ineinander gesteckten, an einer Seite offenen Teilen (20, 22) besteht, so dass das Element in seiner Längs- oder Breitenerstreckung teleskopierbar und somit an die Dimension der aufzubewahrenden Instrumente bzw. Siebkörbe mit Instrumenten flexibel anpassbar ist.

7. Verpackungssystem nach einem der Ansprüche 1 bis 6,
**dadurch gekennzeichnet, dass**
in dem Karton- oder kartonförmigen Element selektiv ausklappbare bzw. einstellbare Begrenzungs- oder Fixierungselemente vorgesehen sind, mittels derer ein Umherrutschen der Instrumente bzw. Siebkörbe innerhalb des Karton- oder kartonförmigen Elements beim Transport vermieden oder vermindert werden kann.

8. Verpackungssystem nach einem der Ansprüche 1 bis 7,
**dadurch gekennzeichnet, dass**
das wenigstens eine vliesartige Flächengebilde (10) an dem Bodenteil (12, 12') wenigstens in einem Teilbereich innenseitig fixiert ist.

9. Verpackungssystem nach Anspruch 8,
**dadurch gekennzeichnet, dass**
ein einziges vliesartiges Flächengebilde (10) vorgesehen ist, das rechteckförmig ausgebildet ist, und vorzugsweise in einem Winkel von etwa 45° zur Ausrichtung des Bodenteils (12, 12') an diesem im Mittenbereich der innenseitigen Bodenfläche fixiert ist.

10. Verpackungssystem nach einem der Ansprüche 1 bis 7,
**dadurch gekennzeichnet, dass**
das vliesartige rechteckförmige Flächengebilde (10) ohne Verbindung mit dem Bodenteil (12, 12') dem Kartonelement oder kartonförmigen Element in Form eines Verpackungssets lose beigefügt ist.

11. Verpackungssystem nach einem der Ansprüche 1 bis 10,
**dadurch gekennzeichnet, dass**
Versiegelungsmittel, insbesondere Klebestreifen, vorgesehen sind, mittels derer ein einmaliges Öffnen des Kartonelements oder kartonförmigen Elements nach Anbringen der Versiegelungsmittel sicher dauerhaft erkannt werden kann.

12. Verpackungssystem nach einem der Ansprüche 1 bis 11,
**dadurch gekennzeichnet, dass**
an dem Kartonelement oder kartonförmigen Element und/oder dem wenigstens einen vliesartigen Flächengebilde (10) Indikatormittel zur dauerhaften Anzeige eines einmal vorgenommenen Sterilisationsvorganges vorgesehen sind.

13. Kartonelement oder kartonförmiges Element,
**dadurch gekennzeichnet, dass**
dieses zur Verwendung bei einem Verpackungssystem nach einem der vorhergehenden Ansprüche ausgebildet ist.

14. Verfahren zum Sterilitätserhalt während der Lagerung und Transport sowie zur aseptischen Präsentation medizinischer Instrumente mittels eines Verpackungssystems,
**gekennzeichnet durch** die Schritte
a) Bereitstellen der noch unsterilisierten medizinischen Instrumente, ggf. in wenigstens einem Siebkorb;
b) Einschlagen der medizinischen Instrumente und/oder des wenigstens einen Siebkorbs in wenigstens ein vliesartiges Flächengebilde (10), so dass diese bzw. dieser vollständig von dem Flächengebilde (10) umhüllt sind,
c) Einpacken der eingeschlagenen medizinischen Instrumente bzw. des wenigstens einen Siebkorbs in ein geschlossenes Kartonelement oder kartonförmiges Element mit einem Bodenteil (12, 12') und einem Deckelteil (14, 14'), wobei das kartonförmige Element aus einem nicht papierbasierten, gleichwohl kartonartig flexiblen Material besteht;
d) Sterilisieren des geschlossenen Kartonelements oder kartonförmigen Elements mit Inhalt;
e) Lagerung des sterilisierten Kartonelements und Transport des sterilisierten Kartonelements oder kartonförmigen Elements zum Einsatzort;
f) am Einsatzort Öffnen des Deckelteils (14, 14') und Eröffnung des vliesartiges Flächengebilde (10) sowie Entnahme der medizinischen Instrumente.
